# EUROPÄISCHE PATENTSCHRIFT

(11) **EP 1 257 525 B1**
(45) Veröffentlichungstag und Bekanntmachung des Hinweises auf die Patenterteilung: **01.02.2006**
(21) Anmeldenummer: 01907532.4
(22) Anmeldetag: 14.02.2001
(51) Int. Cl.: C07C 235/60, C07C 217/20, C01B 21/14

(54) **STABILISATOR FÜR HYDROXYLAMINLÖSUNGEN**
STABILIZING AGENT FOR HYDROXYLAMINE SOLUTIONS
AGENT STABILISANT POUR SOLUTIONS D'HYDROXYLAMINE

(30) Priorität: 22.02.2000 DE 10008080
(43) Veröffentlichungstag der Anmeldung: 20.11.2002
(73) Patentinhaber: BASF Aktiengesellschaft, 67056 Ludwigshafen (DE)
(72) Erfinder: WEBER, Markus, 67061 Ludwigshafen (DE); STERZEL, Hans-Josef, 67125 Dannstadt-Schauernheim (DE); STRÖFER, Eckhard, 68163 Mannheim (DE); WATZENBERGER, Otto, 68199 Mannheim (DE)
(86) Internationale Anmeldenummer: PCT/EP2001/001605
(87) Internationale Veröffentlichungsnummer: WO 2001/062710

(56) Entgegenhaltungen:
- DE-A- 4 025 788
- DE-A- 19 547 759
- JP-A- 51 132 242
- US-A- 4 102 858
- US-A- 4 110 306
- HAJELA, SHARAD ET AL: "A Tris-hydroxymethyl-Substituted Derivative of Gd-TREN-Me-3,2-HOPO: An MRI Relaxation Agent with Improved Efficiency" J. AM. CHEM. SOC., Bd. 122, Nr. 45, 2000, Seiten 11228-11229, XP002171114
- LOCKHOFF, OSWALD: "An access to glycoconjugate libraries through multicomponent reactions" ANGEW. CHEM., INT. ED., Bd. 37, Nr. 24, 1998, Seiten 3436-3439, XP002171115
- VOEGTLE, FRITZ ET AL: "4-(Dimethylamino)-8-hydroxyquinoline as a new chelating ligand and as a donor-reinforced end group in podands" CHEM. BER., Bd. 118, Nr. 4, 1985, Seiten 1556-1563, XP002171116
- VOEGTLE, FRITZ ET AL: "Ligand structure and complexation. XLIV. Synthesis and selectivity of new four-armed noncyclic neutral ligands" CHEM. BER., Bd. 112, Nr. 3, 1979, Seiten 899-907, XP002171117
- VOEGTLE, FRITZ ET AL: "Ligand structure and complexation. 20. Noncyclic cryptates" ANGEW. CHEM., Bd. 89, Nr. 8, 1977, Seiten 564-565, XP002171118

## Beschreibung

Die vorliegende Erfindung betrifft stabilisierte Hydroxylaminlösungen enthaltend mindestens eine Verbindung der Formel (I) oder (II) als Stabilisator, die Verwendung von Verbindungen der Formel (I) oder (II) als Stabilisator in Hydroxylaminlösungen und Verfahren zur Stabilisierung von Hydroxylaminlösungen.

Ferner betrifft die vorliegende Erfindung Tri-(4-(2,3-di-hydroxyphenyl)-4-keto-3-azabutyl)-amin der Formel

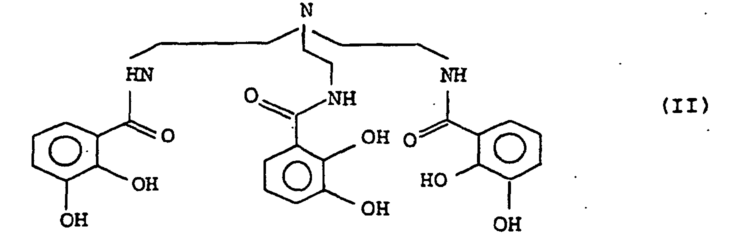

Basische oder neutrale Lösungen, die Hydroxylamin in Form der freien Base enthalten, finden zahlreiche Anwendungen, beispielsweise bei der Synthese von Feinchemikalien oder in der Elektronikindustrie.

Bekannterweise sind solche Lösungen instabil und zersetzen sich zu Ammoniak, Stickstoff, Stickoxiden und Wasser, so daß Transport und Lagerung solcher Lösungen problematisch ist.

Um die Zersetzung zu verlangsamen, setzt man den Lösungen einen Stabilisator zu. Als Stabilisator wurden bereits vorgeschlagen.

Thioglykolsäure (JP-A-58069843), Glycerinmonoether und Ethylenoxidaddukte davon (DE-A-2919554), Hydroxyanthrachinone (DE-A-3343600), Hydroxychinoline (DE-A-3345734), Polyhydroxyhexano-1,4-lacton (DE-A-3345733), Anthocyane (DE-A-3347260), Hydroxychinaldine, Flavone, Benzonitril, N-Phenyl-N-hydroxythioharnstoff (DE-A-3601803), Flavane (DE-A-3343599), Thiosulfate, Mercaptobenzothiazole, Mercaptoalkanole, Mercaptothiazoline, Thiuramdisulfide, Thioharnstoffe (EP-A-516933), das Tetranatriumsalz der Ethylendiamintetraessigsäure, das Trinatriumsalz der N-Hydroxyethylethylendiaminotriessigsäure, Polyvinylpyrrolidon oder Poly-N-vinyl-5-ethyl-2-oxazolidinon (US-A-3145082), Amidoxime (US-A-3480391), Hydroxamsäuren (US-3480391), Hydroxyharnstoffe (US-3,544,270), Dipyridylverbindungen (JP-A-58069842), Aminochinoline (JP-A-58069844), Phenanthroline (JP-A-58069841), und Polyhydroxyphenole (JP-A-4878099).

Nachteilig bei diesen Stabilisatoren ist, daß sie eine Hydroxylaminlösung für eine längere Lagerung nicht ausreichend stabilisieren.

Als Stabilisator, der eine längere Lagerung einer Hydroxylaminlösung ermöglicht, wurde in DE-A-19 547 759 Trans-1,2-Diaminocyclohexan-N,N,N',N'-tetraessigsäure vorgeschlagen.

Dieser Stabilisator ermöglicht zwar eine längere Lagerzeit als die zuvor genannten. Jedoch besteht weiterer Bedarf für eine Verlängerung der möglichen Lagerdauer von Hydroxylaminlösungen.

JP 51/132 242 A offenbart unter Anderem Ester der Nitrilotriessigsäure, der Ethylendiamin-tetraessigsäure und 1,4-Phenylendiamintetraessigsäure. US 4 110 306 lehrt Ester der Nitrilotriessigsäure mit zyklischen Liganden. S. Hajela et al. berichten in J. Am. Chem. Soc. 122 (2000) 11228 - 11229 über die Synthese von an den 2,3-Positionen des Phenylrings oxisubstituierten Tri-(4-(1-Methylpyridin-4-yl)-4-keto-3-aza-2-hydroximethylbutyl)amin und seine Verwendung seines Gadoliniumkomplexes als Relaxationsmittel für bildgebende Magnetresonanzverfahren. O. Lockhoff berichtet in Angew. Chem., Int. Ed. Engl. 37 (1998) 3436 - 3439 über Mehrkomponentenreaktionen zur Synthese von mehrfach glycosylierten Amino- oder Hydroxisäurederivaten. DE 40 25 788 A1 lehrt reduzierende Chelatbildner ihre Technetium- und Rhenium-Komplexe und ihre Verwendung in medizinischer Diagnostik und Therapie und weist darauf hin, dass Tri-(4-(2,3-di-hydroxiphenyl)-4-oxabutyl)-amin als Chelatbildner für Germanium und Silizium aus einer Publikation von B. Wolff in Angew. Chem. 98 (1986) 173 bekannt ist. F. Vögtle et al. berichten in Angew. Chem., Int. Ed. Engl. 16 (1977) 548 - 549 über nichtzyklische Kryptanden, die formal trisubstituierte Amine sind. US 4 102 858 offenbart unter Anderem eine Reihe von vom (2,2,6,6-Tetramethylpiperidin-4-yl)nitrilotriacetat abgeleiteter Verbindungen, die als UV-Stabilisator in Kunstharzen eingesetzt werden.

Der vorliegenden Erfindung liegt daher die Aufgabe zugrunde, einen Stabilisator für Hydroxylaminlösungen bereitzustellen, der eine bessere Möglichkeit zur Stabilisierung, insbesondere Langzeitstabilisierung, von Hydroxylaminlösungen gestattet.

Demgemäß wurden die eingangs definierten stabilisierten Hydroxylaminlösungen enthaltend mindestens eine Verbindung der Formel (I) oder (II) als Stabilisator, die Verwendung von Verbindungen der Formel (I) oder (II) als Stabilisator in Hydroxylaminlösungen, Verfahren zur Stabilisierung von Hydroxylaminlösungen und Verbindungen der Formel (II) gefunden.

Die Verbindungen der Formel (I) oder (II)können in reiner Form oder in Form von Salzen mit organischen Säuren, wie Ameisensäure, Essigsäure, Benzoesäure, oder vorzugsweise anorganischen Säuren, wie Schwefelsäure, Salpetersäure, Phosphorsäure, Phosphoriger Säure, vorzugsweise einer Halogenwasserstoffsäure, insbesondere Chlorwasserstoff oder Bromwasserstoff, eingesetzt werden.

Erfindungsgemäß kommen Tri-(4-(2,3-di- hydroxy-phenyl)-4-oxabutyl)-amin der Formel 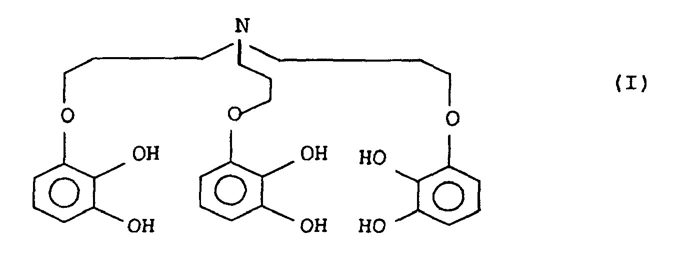 und Tri-(4-(2,3-di-hydroxy-phenyl)-4-keto-3-azabutyl)-amin der Formel 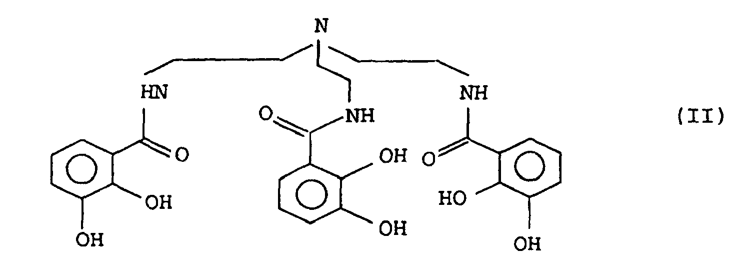 sowie der deren Salze, bevorzugt mit Halogenwasserstoff, insbesondere Chlorwasserstoff oder Bromwasserstoff, wie Tri-(4-(2,3-di-hydroxy-phenyl)-4-keto-3-azabutyl)-amin-hydrochlorid, Tri-(4-(2,3-di-hydroxy-phenyl)-4-keto-3-azabutyl)-amin-hydrobromid, Tri-(4-(2,3-di-hydroxy-phenyl)-4-oxabutyl)-amin-hydrochlorid und Tri-(4-(2,3-di-hydroxy-phenyl)-4-oxabutyl)-amin-hydrobromid in Betracht.

Die erfindungsgemäßen stabilisierten Hydroxylaminlösungen enthalten wenigstens eine, insbesondere 1, Verbindung der Formel (I) oder (II) in freier Form oder in Form eines oder mehrerer zumindest teilweise neutralisierten Salze, d.h. freie Verbindung der Formel (I) oder (II) liegt im Gemisch mit einem oder mehreren Salzen einer oder beider Verbindungen der Formel (I) oder (II) im Gemisch als Stabilisator vor. Ebenso können die erfindungsgemäßen stabilisierten Hydroxylaminlösungen wenigstens eine, insbesondere 1, Verbindung der Formel (I) oder (II) in Form eines oder mehrerer vollständig neutralisierte Salze enthalten.

Die erfindungsgemäßen stabilisierten Hydroxylaminlösungen enthalten Hydroxylamin in Form der freien Base oder ein zumindest teilweise neutralisiertes Hydroxylaminsalz, d.h. freies Hydroxylamin liegt im Gemisch mit dem Hydroxylaminsalz vor.

Die erfindungsgemäß zur Anwendung kommenden Stabilisatoren sind zur Stabilisierung aller Arten von Hydroxylaminlösungen brauchbar. Es kann sich um wäßrige Lösungen oder Lösungen von Hydroxylamin in einem organischen Lösungsmittel, wie Methanol, Ethanol, n-Propanol, Isopropanol, Aceton, Tetrahydrofuran, oder um Gemische von Wasser und organischen Lösungsmitteln handeln. Die erfindungsgemäß zur Anwendung kommenden Stabilisatoren sind in den Lösungsmitteln, in welchen Hydroxylamin löslich ist, ebenfalls löslich.

Hydroxylaminlösungen werden im allgemeinen durch Umsetzung eines Hydroxylammoniumsalzes, insbesondere Hydroxylammoniumsulfat, Hydroxylammoniumchlorid und Hydroxylammoniumphosphat, mit einer geeigneten Base, wie Ammoniak, Natriumhydroxid, Kaliumhydroxid oder Calciumhydroxid, gewonnen. Bei vollständiger Neutralisation des Hydroxylammoniumsalzes erhält man eine Lösung, die freies Hydroxylamin und das Salz enthält, das aus dem Basenkation und dem im Hydroxylammoniumsalz vorhandenen Säureanion stammt. Das Salz kann vollständig oder teilweise abgetrennt werden. Das Hydroxylammoniumsalz kann auch nur teilweise mit der Base neutralisiert werden. Man erhält dann eine Lösung, die neben freiem Hydroxylamin und dem erwähnten Salz auch noch unumgesetztes Hydroxylammoniumsalz enthält. Alle diese Lösungen können erfindungsgemäß stabilisiert werden, wobei die Art des Anions im Hydroxylammoniumsalz nicht kritisch ist.

Die erfindungsgemäßen stabilisierten Hydroxylaminlösungen enthalten die Verbindungen der Formel (I) oder (II)in einer zur Stabilisierung ausreichenden Menge. Vorzugsweise enthalten sie 0,001 bis 20 Gew.-% (10 bis 200 000 ppm), insbesondere 0.001 bis 10 Gew.-%, besonders bevorzugt 0,01 bis 5 Gew.-%, ganz besonders bevorzugt 0,02 - 2 Gew.-% Verbindung der Formel (I) oder (II), bezogen auf den Hydroxylamingehalt. Die Hydroxylaminkonzentration beträgt im allgemeinen 1 - 100 Gew.-%, insbesondere 1 bis 70 Gew.-%, bezogen auf das Gesamtgewicht der Lösung.

Der Stabilisator kann vor oder nach Neutralisation des Hydroxylaminsalzes, bevorzugt aber vor der Neutralisation, zugesetzt werden.

Die Verbindungen der Formel (I) oder (II) sind in einem weiten Temperaturbereich wirksam. So stabilisieren sie Hydroxylaminlösungen im Bereich von -20°C bis 130°C, vorzugsweise bei -10°C bis 100°C. Sie sind aber auch bei deutlich höheren Temperaturen unter den zur Verflüssigung der Lösung erforderlichen Drücken als Stabilisator geeignet.

Die nachfolgenden Beispiele erläutern die Erfindung, ohne sie zu begrenzen. Die im Rahmen der Beispiele vorgenommene Bestimmung der Hydroxylamin-Konzentrationen erfolgt durch Redoxtitration.

### Beispiele:

In einem 50 ml Rundkolben aus Glas wurden jeweils 20 ml 50 %-ige wäßrige Hydroxylaminlösung vorgelegt und mit 300 ppm (m/m) Stabilisator gemäß Tabelle 1, bezogen auf die Masse an Hydroxylamin, versetzt. Der Rundkolben wurde auf Raumtemperatur gehalten, bzw. mit einem Ölbad auf 100°C erwärmt und bei dieser Temperatur gehalten. Die Hydroxylamingehalte der stabilisierten Lösungen bei 100°C wurden nach 15 Stunden durch Redoxtitration von Proben bestimmt. Dazu wurden aus den Lösungen definierte Probenmengen gezogen, mit einem Überschuß an schwefelsaurer Ammoniumeisen (III) -salzlösung 5 Minuten gekocht und das gebildete Eisen (II)-salz mit Cer (IV)-salzlösung zurücktitriert. Der Umschlagspunkt wurde potentiometrisch bestimmt.

Die Rundkolben wurden so lange bei 100°C gehalten, bis eine rapide Zersetzung des Hydroxylamins einsetzte. Zu diesem Zeitpunkt ist der Stabilisator so weit abgebaut, daß er keinen ausreichenden Schutz vor der Zersetzung des Hydroxylamins bietet.

Aus der Tabelle 1 ist ersichtlich, daß die Verbindungen der Formel (I) oder (II) Hydroxylamin-Lösungen am längsten stabilisieren können.

**Tabelle 1**

| Zusatz | Hydroxylamin-Gehalt der Lösung [%] nach 15 h | Zeit bis zur Zersetzung [h] |
|---|---|---|
| Kein Zusatz | 25 | - - |
| Thioacetamid | 49,5 | 40 |
| Thioharnstoff | 49,5 | 40 |
| Thiouracil | 48,9 | 20 |
| Trithiocyanursäure | 49,4 | 22 |
| Trans-1,2-Diaminocyclo-hexan-N,N,N' ,N'-tetraessigsäure | 49,7 | 75 |
| Verb. Formel (I) als Chlorid | 49,7 | > 100 |
| Verb. Formel (II) als Bromid | 49, 8 | > 100 |

## Patentansprüche

1. Stabilisierte Hydroxylaminlösungen, die als Stabilisator wenigstens eine Verbindung, ausgewählt aus der Gruppe bestehend aus Tri-(4-(2,3-di-hydroxy-phenyl)-4-oxabutyl)-amin, Tri-(4-(2,3-di-hydroxy-phenyl)-4-keto-3-azabutyl)-amin oder deren Salzen enthalten.

2. Stabilisierte Hydroxylaminlösungen nach Anspruch 1, die 0,001 bis 20 Gew.-%, insbesondere 0,001 bis 10 Gew.-%, vorzugsweise 0,01 bis 5 Gew.-%, besonders bevorzugt 0,02 bis 2 Gew.-% bezogen auf Hydroxylamin, wenigstens einer Verbindung, ausgewählt aus der Gruppe bestehend aus Tri-(4-(2,3-di-hydroxyphenyl)-4-oxabutyl)-amin, Tri-(4-(2,3-di-hydroxy-phenyl)-4-keto-3-azabutyl)-amin oder deren Salzen enthalten.

3. Verwendung von mindestens einer Verbindung, ausgewählt aus der Gruppe bestehend aus Tri-(4-(2,3-di-hydroxy-phenyl)-4-oxabutyl)-amin, Tri-E4-(2,3-di-hydroxy-phenyl)-4-keto-3-azabutyl)-amin oder deren Salzen zur Stabilisierung von Hydroxylaminlösungen.

4. Verfahren zur Stabilisierung einer Hydroxylaminlösung, **dadurch gekennzeichnet, daß** man eine Verbindung, ausgewählt aus der Gruppe bestehend aus Tri-(4-(2,3-di-hydroxy-phenyl)-4-oxabutyl)-amin, Tri-(4-(2,3-di-hydroxy-phenyl)-4-keto-3-azabutyl)-amin oder deren Salzen und eine Hydroxylaminlösung zusammengibt.

5. Tri-(4-(2,3-di-hydroxy-phenyl)-4-keto-3-azabutyl)-amin und dessen Salze.

## Claims

1. A stabilized hydroxylamine solution comprising as stabilizer at least one compound selected from the group consisting of tri(4-(2,3-dihydroxyphenyl)-4-oxabutyl)amine, tri(4-(2,3-dihydroxyphenyl)-4-keto-3-azabutyl)amine, and salts thereof.

2. The stabilized hydroxylamine solution according to claim 1 comprising from 0.001 to 20% by weight, in particular from 0.001 to 10% by weight, preferably from 0.01 to 5% by weight, with particular preference from 0.02 to 2% by weight, based on hydroxylamine, of at least one compound selected from the group consisting of tri(4-(2,3-dihydroxyphenyl)-4-oxabutyl)amine, tri(4 -(2,3-dihydroxyphenyl)-4-keto-3-azabutyl)amine, and salts thereof.

3. The use of at least one compound selected from the group consisting of tri(4-(2,3-dihydroxyphenyl)-4-oxabutyl)amine, tri(4-(2,3-dihydroxyphenyl)-4-keto-3-azabutyl)amine, and salts thereof for stabilizing hydroxylamine solutions.

4. A method of stabilizing a hydroxylamine solution, which comprises bringing a compound selected from the group consisting of tri(4-(2,3-dihydroxyphenyl)-4-oxabutyl)amine, tri(4-(2,3-dihydroxyphenyl)-4-keto-3-azabutyl)amine, and salts thereof together with a hydroxylamine solution.

5. Tri(4-(2,3-dihydroxyphenyl)-4-keto-3-azabutyl)amine or a salt thereof.

## Revendications

1. Solutions d'hydroxylamine stabilisées qui contiennent, comme agent stabilisant, au moins un composé choisi parmi le groupe constitué de tri-(4-(2,3-di-hydroxy-phényl)-4-oxabutyl)-amine, de tri-(4-(2,3-di-hydroxy-phényl)-4-céto-3-azabutyl)-amine ou de leurs sels.

2. Solutions d'hydroxylamine stabilisées suivant la revendication 1, qui contiennent 0,001 à 20 % en poids, en particulier 0,001 à 10 % en poids, de préférence 0,01 à 5 % en poids, particulièrement avantageusement 0,02 à 2 % en poids, par rapport à l'hydroxylamine, d'au moins un composé choisi parmi le groupe constitué de tri-(4-(2,3-di-hydroxy-phényl)-4-oxabutyl)-amine, de tri-(4-(2,3-di-hydroxy-phényl)-4-céto-3-azabutyl)-amine ou de leurs sels.

3. Utilisation d'au moins un composé choisi parmi le groupe constitué de tri-(4-(2,3-di-hydroxyphényl)-4-oxabutyl)-amine, de tri-(4-(2,3-dihydroxy-phényl)-4-céto-3-azabutyl)-amine ou de leurs sels, pour la stabilisation de solutions d'hydroxylamine.

4. Procédé de stabilisation d'une solution d'hydroxylamine, **caractérisé en ce qu'**on réunit un composé choisi parmi le groupe constitué de tri-(4-(2,3-di-hydroxy-phényl)-4-oxabutyl)-amine, de tri-(4-(2,3-di-hydroxy-phényl)-4-céto-3-azabutyl)-amine ou de leurs sels et une solution d'hydroxylamine.

5. Tri-(4-(2,3-di-hydroxy-phényl)-4-céto-3-azabutyl)-amine et ses sels.
